# EUROPÄISCHE PATENTSCHRIFT

(11) **EP 0 768 310 B1**
(45) Veröffentlichungstag und Bekanntmachung des Hinweises auf die Patenterteilung: **15.12.1999**
(21) Anmeldenummer: 96115949.8
(22) Anmeldetag: 04.10.1996
(51) Int. Cl.: C07D 487/04, C07D 243/00, C07D 235/00

(54) **Verfahren zur Herstellung von Imidazo-Benzodiazepin-Derivaten**
Process for the production of imidazo-benzodiazepine derivatives
Procédé pour la préparation de dérivés de l'imidazo-benzodiazépine

(30) Priorität: 10.10.1995 CH 285495
(43) Veröffentlichungstag der Anmeldung: 16.04.1997
(73) Patentinhaber: F. HOFFMANN-LA ROCHE AG, 4070 Basel (CH)
(72) Erfinder: Bender, Karl-Heinz, 79395 Neuenburg (DE); Breuninger, Manfred, 79713 Bad Säckingen (DE); Froom, Manfred, 79112 Freiburg (DE); Schmitt, Siegfried, 4153 Reinach (CH); Steiner, Kurt, 4656 Starrkirch-Wil (CH)
(74) Vertreter: Poppe, Regina

(56) Entgegenhaltungen:
- CH-A- 632 511
- US-A- 4 280 957
- US-A- 4 307 237

## Beschreibung

Die vorliegende Erfindung betrifft ein neues Verfahren zur Herstellung von 4H-Imidazo[1,5.a][1,4]benzodiazepinderivaten der allgemeinen Formel I 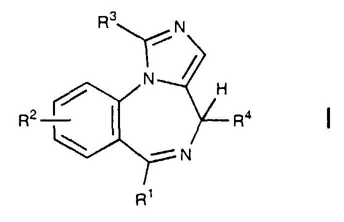 worin
- R¹: Phenyl, durch Halogen oder Nitro monosubstituiertes Phenyl;
- R²: Wasserstoff, Halogen, Nitro, Cyano, Trifluormethyl oder niederes Alkyl;
- R³: Wasserstoff oder niederes Alkyl;
- R⁴: Wasserstoff oder niederes Alkyl bedeuten
und von pharmazeutisch anwendbaren Salzen dieser Verbindungen durch Decarboxylierung der entsprechenden 4H-Imidazo[1,5.a][1,4]benzodiazepincarbonsäure der allgemeinen Formel II:

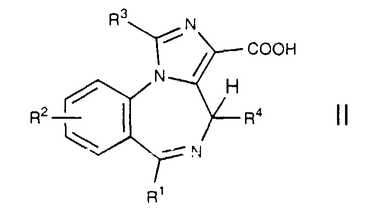

Die Schweizer Patentschrift 632 511 und die US Patentschriften 4,280,957 und 4,307,237 beschreiben ein Verfahren zur Herstellung von Imidazo-Benzodiazepin-Derivaten der allgemeinen Formel I durch Decarboxylierung der entsprechenden Imidazo-Benzodiazepin-Carbonsäure bei 100-350°C, gegebenenfalls in Gegenwart eines Lösungsmittels und eines Katalysators. Dieses Verfahren hat den Nachteil, dass neben dem gewünschten 4H-Imidazo[1,5-a][1,4]benzodiazepinderivat der Formel I eine erhebliche Menge des entsprechenden isomeren 6H-Imidazo[1,5-a][1,4]benzodiazepinderivats entsteht, was eine aufwendige Nachisomerisierung bedingt.

Das erfindungsgemässe Verfahren ist dadurch gekennzeichnet, dass die oben erwähnte Decarboxylierung bei erhöhter Temperatur und unter erhöhtem Druck in Gegenwart eines organischen Lösungsmittels decarboxyliert.

Der Ausdruck "niederes Alkyl" umfasst geradkettige oder verzweigte gesättigte Kohlenwasserstoffreste mit bis zu 7 Kohlenstoffatomen, wie Methyl, Ethyl, n-Propyl, Isopropyl, n-Butyl, sek.Butyl, Isobutyl, n-Pentyl, n-Hexyl und dergleichen.

Der Ausdruck "Halogen" umfasst Chlor, Brom, Fluor und Jod.

Der durch R¹ bezeichnete Phenylrest kann mono- oder di-substituiert sein. Geeignete Monosubstituenten umfassen Halogen und Nitro, vorzugsweise in 2-Stellung des Phenylrings. Bevorzugt ist ein Fluor-Substituent in 2-Stellung des Phenylrings.

Im Falle, dass R⁴ niederes Alkyl bedeutet, tritt optische Isomerie auf, und sowohl die optischen Antipoden als auch Racemate werden von der vorliegenden Erfindung umfasst.

Der Ausdruck "pharmazeutisch anwendbares Salz" umfasst Salze mit anorganischen und organischen pharmazeutisch anwendbaren Säuren wie Salzsäure, Schwefelsäure, Phosphorsäure, Citronensäure, Ameisensäure, Maleinsäure, Essigsäure, Bernsteinsäure, Weinsäure, p-Toluolsulfonsäure und dergleichen.

Die Decarboxylierung erfolgt in einem Temperaturbereich von 150° C - 350° C , vorzugsweise bei 250° C - 350°C. Besonders bevorzugt sind 280-290° C.

Erfindungsgemäss wird unter erhöhtem Druck gearbeitet, in einem Druckbereich von 50-150 bar, vorzugsweise bei 90-110 bar.

Das Verfahren kann sowohl als batch-Verfahren als auch als kontinuierliches Verfahren geführt werden. Bevorzugt ist eine kontinuierliche Verfahrensführung.

Geeignete Lösungsmittel zur Decarboxylierung der Verbindungen der allgemeinen Formel II im Rahmen der vorliegenden Erfindung sind n-Butanol, n-Butylacetat, Essigester, Ethanol, Ethylenglycol, Hexan, Isopropylacetat, n-Propanol, Tetralin und Toluol. Bevorzugt ist n-Butanol.

Die Menge an Lösungsmittel ist nicht kritisch.

Das Rohprodukt besteht aus den beiden isomeren Derivaten 4H-Imidazo[1,5-a][1,4]benzodiazepin und 6H-Imidazo[1,5-a][1,4]benzodiazepin. Der Gehalt an 4H-Imidazo[1,5-a][1,4]benzodiazepinderivat beträgt circa 85%.

Die Aufarbeitung des Rohproduktes kann durch Umkristallisation aus einem geeigneten Lösungsmittel erfolgen. Für die Umkristallisation geeignete Lösungsmittel sind n-Butanol, Butylacetat, t.-Butylmethylether, Ethanol, Ethylacetat, Isopropylacetat und Gemische dieser Lösungsmittel. Bevorzugt ist Isopropylacetat. Das gewünschte 4H-Imidazo[1,5-a][1,4)-benzodiazepinderivat kristallisiert aus, während das polarere 6H-Imidazo[1,5-a] [1,4]benzodiazepinderivat in Lösung bleibt und abgetrennt werden kann.

Erwünschtenfalls kann das Kristallisat durch nochmaliges Umkristallisieren, beispielsweise aus Ethanol, nachgereinigt werden.

Der Gehalt an 6H-Imidazo[1,5-a][1,4]benzodiazepinderivat ist nach der Reinigung kleiner als 0,2%.

Die Mutterlaugen der Kristallisationen können aufkonzentriert werden, wobei ein Gemisch von 4H-Imidazo[1,5-a][1,4]benzodiazepinderivat und 6H-Imidazo[1,5-a][1,4]benzodiazepinderivat im Isomerenverhältnis zwischen 70:30 und 50:50 erhalten wird. Das Isomerenverhältnis kann durch Zugabe von KOH beeinflusst werden. Dazu wird zweckmässigerweise der aus der Mutterlauge gewonnene Kristallkuchen mit fester KOH in einem geeigneten Lösungsmittel, beispielsweise in n-Butanol, mindestens 12 Stunden suspensiert. Das Isomerenverhältnis beträgt dann circa 95:5. Zur weiteren Aufarbeitung kann nach Abtrennung der KOH, wie oben beschrieben, umkristallisiert werden.

Die Verbindungen der Formel I und ihre pharmazeutisch anwendbaren Säureadditionssalze sind bekannte Muskelrelaxantien, Sedativa und Antikonvulsiva.

Am folgenden Beispiel einer kontinuierlichen Hochdruckdecarboxylierung von 8-Chlor-6-(2-fluor-phenyl)-1-methyl-4H-imidazo-[1,5a] [1,4]benzodiazepin-3-carbonsäure wird die Erfindung näher erläutert.

### Beispiel

### Herstellung von 8-Chlor-6-(2-fluor-phenyl)-1-methyl-4H-imidazo[1,5a][1,4]-benzodiazepin.

Das kontinuierlich geführte Verfahren umfasst die folgenden Schritte:
a) Decarboxylierung
   8-Chlor-6-(2-fluor-phenyl)-1-methyl-4H-imidazo[1,5a][1,4]benzodiazepin-3-carbonsäure wird in Form einer 50%igen Suspension in n-Butanol auf den kontinuierlichen Reaktor gegeben. Die Decarboxylierung erfolgt bei circa 280°C und 100 bar. Die Verweilzeit im Reaktor beträgt 2-4 Minuten, wobei ein Umsatz von mehr als 99% erreicht wird. Das Produktgemisch besteht aus circa 85% 8-Chlor-6-(2-fluor-phenyl)-1-methyl-4H-imidazo[1,5a][1,4]benzodiazepin und 15% 8-Chlor-6-(2-fluor-phenyl)-1-methyl-6H-imidazo[1,5a][1,4]benzodiazepin.
b) Aufarbeitung des Rohprodukts
   Das n-Butanol wird abgedampft bis zu einem Gehalt von circa 10 %.
   Zweimalige Umkristallisation aus Isopropylacetat ergibt nahezu reines 8-Chlor-6-(2-fluor-phenyl)-1-methyl-4H-imidazo[1,5a][1,4]benzodiazepin. Der Gehalt an 8-Chlor-6-(2-fluor-phenyl)-1-methyl-6H-imidazo[1,5a][1,4]benzodiazepin liegt unter 0,2%.
c) Nachreinigung
   Das Produkt ist leicht gelb gefärbt und wird aus Ethanol umkristallisiert.
   Die Ausbeute nach Aufarbeitung des Rohprodukts und Nachreinigung beträgt 45-55%.
d) Aufarbeitung der Mutterlauge
   Die Mutterlaugen der Kristallisationen aus Isopropylacetat und aus Ethanol werden vereinigt und stark aufkonzentriert. Das Gemisch aus 8-Chlor-6-(2-fluor-phenyl)-1-methyl-4H-imidazo[1,5a][1,4]benzodiazepin und 8-Chlor-6-(2-fluor-phenyl)1-methyl-6H-imidazo[1,5a][1,4]benzodiazepin kristallisiert nahezu quantitativ aus. Das Isomerenverhältnis liegt zwischen 70:30 und 50:50. Das Isomerengemisch wird zur Nachisomerisierung in n-Butanol unter Zusatz von fester KOH mindestens 12 Stunden bei Raumtemperatur suspendiert. Danach wird das Kristallisat abgetrennt und mehrmals in Wasser digeriert, bis das KOH quantitativ aus dem Kristallisat entfernt ist. Das feuchte Kristallisat, welches jetzt ein Isomerenverhältnis von circa 95:5 aufweist, wird zweimal aus Isopropylacetat umkristallisiert. Restwasser wird als Azeotrop entfernt. Die Nachreinigung erfolgt durch Umkristallisation aus Ethanol.
   Ausbeute 15-25%

## Patentansprüche

1. Verfahren zur Herstellung von 4H-Imidazo[1,5-a][1,4]benzodiazepin-derivaten der allgemeinen Formel I 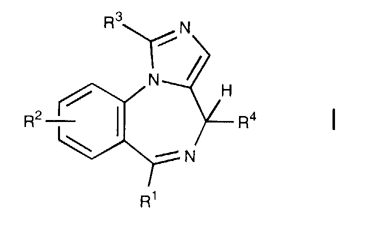 worin
R¹ Phenyl, durch Halogen oder Nitro monosubstituiertes Phenyl;
R² Wasserstoff, Halogen, Nitro, Cyano, Trifluormethyl oder C₁-C₇-Alkyl;
R³ Wasserstoff oder C₁-C₇-Alkyl ;
R⁴ Wasserstoff oder C₁-C₇-Alkyl bedeuten
und von pharmazeutisch anwendbaren Salzen dieser Verbindungen, durch Decarboxilierung einer 4H-Imidazo[1,5-a][1,4]benzodiazepin-carbonsäure der allgemeinen Formel II 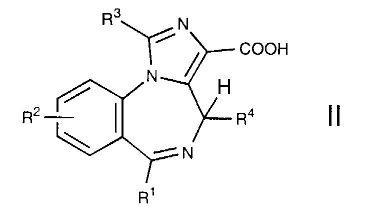 worin R¹, R², R³ und R⁴ die obigen Bedeutungen besitzen, dadurch gekennzeichnet, dass die Decarboxylierung bei einer Temperatur zwischen 150°C und 350°C und unter einem Druck zwischen 50 und 150 bar in Gegenwart eines organischen Lösungsmittels stattfindet.

2. Verfahren nach Anspruch 1, dadurch gekennzeichnet, dass 8-Chlor-6-(2-fluor-phenyl)-1-methyl-4H-imidazo[1,5a][1,4]benzodiazepin hergestellt wird.

3. Verfahren nach Anspruch 1 oder 2, dadurch gekennzeichnet, dass die Temperatur 250° C - 350°C beträgt.

4. Verfahren nach Anspruch 1, 2 oder 3, dadurch gekennzeichnet, dass der Druck 90-110 bar beträgt.

5. Verfahren nach einem der Ansprüche 1-4, dadurch gekennzeichnet, dass die Decarboxylierung in Gegenwart von n-Butanol, n-Butylacetat, Essigester, Ethanol, Ethylenglycol, Hexan, Isopropylacetat, n-Propanol, Tetralin oder Toluol durchgeführt wird.

6. Verfahren nach Anspruch 5, dadurch gekennzeichnet, dass die Decarboxylierung in n-Butanol durchgeführt wird.

7. Verfahren nach einem der Ansprüche 1-6, dadurch gekennzeichnet, dass das Verfahren als kontinuierliches Verfahren geführt wird.

8. Verfahren nach einem der Ansprüche 1-7, dadurch gekennzeichnet, dass das erhaltene Produkt zur Reinigung aus n-Butanol, Butylacetat, t.-Butylmethylether, Ethanol, Ethylacetat oder Isopropylacetat umkristallisiert wird.

9. Verfahren nach Anspruch 8, dadurch gekennzeichnet, dass die Umkrisatallisation aus Isopropylacetat erfolgt.

10. Verfahren nach Anspruch 8 oder 9, dadurch gekennzeichnet, dass das durch Umkristallisation gereinigte Produkt durch nochmaliges Umkristallisieren nachgereinigt wird.

11. Verfahren nach Anspruch 10, dadurch gekennzeichnet, dass das nochmalige Umkristallisieren aus Ethanol erfolgt.

## Claims

1. A process for the manufacture of 4H-imidazo[1,5-a]-[1,4]benzodiazepine derivatives of general formula I 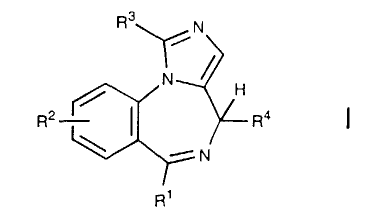 wherein
R¹ signifies phenyl, halogen or nitro monosubstituted phenyl;
R² signifies hydrogen, halogen, nitro, cyano, trifluoromethyl or C₁-C₇-alkyl;
R³ signifies hydrogen or C₁-C₇-alkyl; and
R⁴ signifies hydrogen or C₁-C₇-alkyl
and of pharmaceutically usable salts of these compounds, which process comprises decarboxylating a 4H-imidazo[1,5-a][1,4]-benzodiazepinecarboxylic acid of general formula II 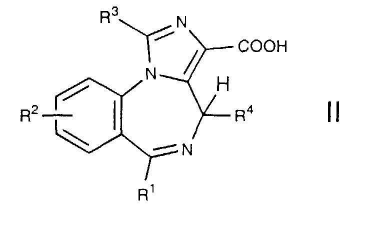 wherein R¹, R², R³ and R⁴ have the above significances, at elevated temperature and at a pressure of 50-150 bar in the presence of an organic solvent.

2. A process according to claim 1, wherein 8-chloro-6-(2-fluorophenyl)-1-methyl-4H-imidazo[1,5a][1,4]benzodiazepine is manufactured.

3. A process according to claim 1 or 2, wherein the temperature is 250°C-350°C.

4. A process according to claims 1, 2 or 3, wherein the pressure is 90-110 bar.

5. A process according to any one of claims 1-4, wherein the decarboxylation is carried out in the presence of n-butanol, n-butyl acetate, ethyl acetate, ethanol, ethylene glycol, hexane, isopropyl acetate, n-propanol, tetralin or toluene.

6. A process according to claim 5, wherein the decarboxylation is carried out in n-butanol.

7. A process according to any one of claims 1-6, which is carried out as a continuous process.

8. A process according to any one of claims 1-7, wherein the product obtained is purified by recrystallization from n-butanol, butyl acetate, t-butyl methyl ether, ethanol, ethyl acetate or isopropyl acetate.

9. A process according to claim 8, wherein the recrystallization is effected from isopropyl acetate.

10. A process according to claim 8 or claim 9, wherein the product which is purified by recrystallization is further purified by repeated recrystallization.

11. A process according to claim 10, wherein the repeated recrystallization is effected from ethanol.

## Revendications

1. Procédé de préparation de dérivés de
4H-imidazo[1,5-a][1,4]benzodiazépine de formule générale I 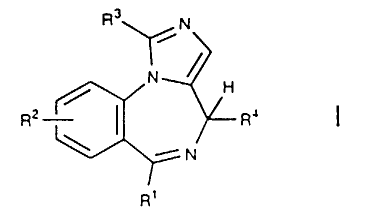 dans laquelle
R¹ représente un phényle, un phényle monosubstitué par un halogène ou un nitro ;
R² représente un hydrogène, un halogène, un nitro, un cyano, un trifluorométhyle ou un alkyle en C₁ à C₇ ;
R³ représente un hydrogène ou un alkyle en C₁ à C₇ ;
R⁴ représente un hydrogène ou un alkyle en C₁ à C₇ ;
et de sels pharmaceutiquement acceptables de ces composés, par décarboxylation d'un acide 4H-imidazo[1,5-a][1,4]benzodiazépine-carboxylique de formule générale II 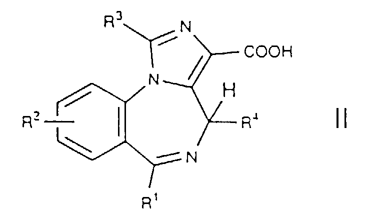 dans laquelle R¹, R², R³ et R⁴ ont les significations données ci-dessus, caractérisé en ce que la décarboxylation s'effectue à une température comprise entre 150°C et 350°C et sous une pression comprise entre 50 et 150 bars en présence d'un solvant organique.

2. Procédé selon la revendication 1, caractérisé en ce qu'on prépare la 8-chloro-6-(2-fluoro-phényl)-1-méthyl-4H-imidazo[1,5a][1,4]benzodiazépine.

3. Procédé selon la revendication 1 ou 2, caractérisé en ce que la température s'élève à 250°C-350°C.

4. Procédé selon la revendication 1, 2 ou 3, caractérisé en ce que la pression s'élève à 90-110 bars.

5. Procédé selon l'une des revendications 1-4, caractérisé en ce que la décarboxylation s'effectue en présence de n-butanol, d'acétate de n-butyle, d'acétate d'éthyle, d'éthanol, d'éthylèneglycol, d'hexane, d'acétate d'isopropyle, de n-propanol, de tétraline ou de toluène.

6. Procédé selon la revendication 5, caractérisé en ce que la décarboxylation s'effectue dans le n-butanol.

7. Procédé selon l'une des revendications 1-6, caractérisé en ce que le procédé est conduit sous forme de procédé continu.

8. Procédé selon l'une des revendications 1-7, caractérisé en ce qu'aux fins de purification on recristallise le produit obtenu à partir du n-butanol, de l'acétate de butyle, de l'éther t.-butylméthylique, de l'éthanol, de l'acétate d'éthyle ou de l'acétate d'isopropyle.

9. Procédé selon la revendication 8, caractérisé en ce que la recristallisation s'effectue à partir de l'acétate d'isopropyle.

10. Procédé selon la revendication 8 ou 9, caractérisé en ce qu'on purifie encore une fois par recristallisation le produit purifié par recristallisation.

11. Procédé selon la revendication 10, caractérisé en ce que la nouvelle recristallisation s'effectue à partir de l'éthanol.
